(19)

Europäisches
Patentamt

European
Patent Office

Office européen
des brevets

(11)     **EP 4 696 230 A1**

(12)     **EUROPEAN PATENT APPLICATION**

(43) Date of publication:
     **18.02.2026  Bulletin 2026/08**

(21) Application number: **25195135.6**

(22) Date of filing: **11.08.2025**

(51) International Patent Classification (IPC):
     ***A61B 5/0245*** (2006.01)    ***A61B 5/11*** (2006.01)
     ***A61B 5/318*** (2021.01)    ***A61B 5/349*** (2021.01)
     ***A61B 5/00*** (2006.01)    ***G16H 20/30*** (2018.01)

(52) Cooperative Patent Classification (CPC):
     **A61B 5/1118; A61B 5/0245; A61B 5/318;**
     **A61B 5/349; A61B 5/7275; G16H 20/30;**
     A61B 5/6823; A61B 2562/0219

(84) Designated Contracting States:
     **AL AT BE BG CH CY CZ DE DK EE ES FI FR GB**
     **GR HR HU IE IS IT LI LT LU LV MC ME MK MT NL**
     **NO PL PT RO RS SE SI SK SM TR**
     Designated Extension States:
     **BA**
     Designated Validation States:
     **GE KH LA MA MD TN**

(30) Priority:  **12.08.2024  US 202418800375**

(71) Applicant: **Medidata Solutions, Inc.**
     **New York, NY 10014 (US)**

(72) Inventors:
     • **AGARWAL, Nishit**
       **New York, NY, 10014 (US)**
     • **CERUOLO, Melissa**
       **New York, NY, 10014 (US)**
     • **ALPHONSE, Sebastian Anand**
       **New York, NY, 10014 (US)**
     • **MEYER, Brett**
       **New York, NY, 10014 (US)**

(74) Representative: **Fish & Richardson P.C.**
     **Highlight Business Towers**
     **Mies-van-der-Rohe-Straße 8**
     **80807 München (DE)**

(54)    **SYSTEMS AND TECHNIQUES FOR MONITORING A USER'S MEDICAL CONDITION USING PHYSIOLOGICAL SENSORS**

(57)     An example medical monitoring system includes a sensor apparatus having cardiac sensors and acceleration sensors, and an electronic device communicatively coupled to the sensor apparatus. The electronic device is configured to cause the sensor apparatus to obtain cardiac data regarding the user during a first period of time, and acceleration data regarding the user during the first period of time. Further, the electronic device is configured to determine, based on the data, first physiological data representing a cardiac activity of the user during the first period of time and second physiological data representing a variation of the acceleration data during the period of time. Further, the electronic device is configured to determine, based on the first physiological data and the second physiological data, a biomarker representing a health of the user, and store a data structure representing the biomarker.

FIG. 1

EP 4 696 230 A1

**Description**

TECHNICAL FIELD

This description generally relates to systems and methods for monitoring a user's medical condition using physiological sensors.

BACKGROUND

In general, a user's health can be assessed by measuring one or more physiological characteristics of the user, and comparing the measured physiological characteristics to a standard. For example, a user having physiological characteristics that meet or exceed a particular standard may be in good health, whereas a user having physiological characteristics that do not meet the standard may be in poor health,

SUMMARY

In general, a medical monitoring system can be used to monitor a user's health and to facilitate treatment of the user.

In an example implementation, a medical monitoring system includes a sensor apparatus configured to obtain sensor data representing one or more physiological characteristics of a user, and one or more processor modules (e.g., computer processors) configured to process the sensor data and determine one or more biomarkers representing the user's medical condition.

For instance, the medical monitoring system can obtain sensor data while the user is exerting physical effort, such as while the user is continuously walking for a period of time (e.g., six minutes). The sensor data can include measurements of the user's cardiac activity, such as the user's heartbeats and/or heart rate variation (HRV) (e.g., obtaining one or more cardiac sensors) during the period of time. Further the sensor data can include measurements of the user's movements (e.g., acceleration data obtained using one or more acceleration sensors) during the period of time. Based on these measurements, the medical monitoring system can determine a biomarker that represents the user's physical health, such as the user's functional capacity and/or cardiopulmonary condition. Further, the medical monitoring system can present the biomarker to the user or another user (e.g., a health care provider) to facilitate treatment of the user and/or monitoring of the user's health over time.

In some implementations, the biomarker can be determined, at least in part, by determining a ratio between (i) a number of heartbeats by the user over the period of time (e.g., 6 minutes), and (ii) a motion metric representing a variation in the movements of the user over that period of time. Further, the value of the biomarker can represent the relative health of the user. For example, over a particular range of values of the biomarker, a lower value can indicate that the user health is relatively high, whereas a higher value for the biomarker can indicate that the user health is relatively low.

Further, in at least some implementations, the biomarker obtained by the medical monitoring system can be used as an estimate or approximation of other biomarkers that represent a user's health that would otherwise require manual input from the user or third-party observer to calculate.

For example, in a traditional "six-minute walk test" (6MWT), a user walks continuously during a six minute period of time. Upon the completion of the period of time, an observer (e.g., a health care provider in a clinical setting) measures the distance that the user has walked during the period of time, and uses the measured distance as a biomarker representing the user's health. For example, if the user walked a long distance during the period of time, the biomarker is greater in value and represents that the user's health is relatively high. If the user walked a short distance during the period of time, the biomarker is lower in value and represents that the user's health is relatively low.

However, the traditional "six-minute walk test" may be difficult to perform in practice, and may be subject to variability. For example, a trained observer may not be readily available to conduct the test. In the absence of a trained observer, the user may conduct the test themselves incorrectly (e.g., measure the distance incorrectly, walk for the incorrect amount of time, etc.), which may negatively affect the accuracy and reliability of the results.

In contrast, the biomarker obtained by the medical monitoring system described herein can approximate the biomarker generated by the "six minute walk test," but without requiring a trained observer or manual input from the user themselves. Accordingly, the user can use the medical monitoring system to monitor their health more easily and accurately (e.g., in a home environment, without the supervision of a health care provider).

The implementations described herein can provide various technical benefits. As an example, the implementations described herein allow a computer system to automatically determine a user's health based on sensor data (e.g., cardiac data, acceleration data, etc.), without requiring manual feedback from a human. Further, the medical monitoring system can provide this functionality by performing computer-specific operations on input data in an objective manner (and in a manner that is not feasible for a human to perform), rather than relying on a subjective human interpretation of the input data which may be less suitable for performance by a computer.

As another example, the implementations described herein can be used to facilitate monitoring and treatment of a user, thereby improving the user's health. For example, a medical monitoring system can generate a biomarker representing the user's health, and provide the biomarker to the user or another user (e.g., a health care provider). Based on this information, the user or other user can take active steps to improve the user's health, such as conducting further tests to diagnose the user's medical condition, changing the user's behavior, lifestyle, and/or diet, or any other action.

In an aspect, a medical monitoring system includes: a sensor apparatus configured to be attached to a user's body, where the sensor apparatus includes: one or more cardiac sensors, and one or more acceleration sensors; an electronic device communicatively coupled to the sensor apparatus, where the electronic device includes one or more computer processors that are configured to: cause the sensor apparatus to obtain, using the one or more cardiac sensors, cardiac data regarding the user during a first period of time; cause the sensor apparatus to obtain, using the one or more acceleration sensors, acceleration data regarding the user during the first period of time; determine, based on the cardiac data, first physiological data representing a cardiac activity of the user during the first period of time; determine, based on the acceleration data, second physiological data representing a variation of the acceleration data during the period of time; determine, based on the first physiological data and the second physiological data, a biomarker representing a health of the user; and store a data structure representing the biomarker.

Implementations of this aspect can include or more of the following features.

In some implementations, the biomarker can represent a functional capacity of the user.

In some implementations, the biomarker can represent a cardiopulmonary condition of the user.

In some implementations, the sensor apparatus can be configured to be attached to the user's chest.

In some implementations, the first period of time can be six minutes.

In some implementations, the one or more cardiac sensors can include an electrocardiogram (ECG) sensor.

In some implementations, the first physiological data can represent a number of heartbeats of the user during the first period of time.

In some implementations, determining the first physiological data can include segmenting the cardiac data into a plurality of cardiac data segments, where each of the cardiac data segments represents a different respective heartbeat of the user.

In some implementations, determining the first physiological data can include determining, based on the cardiac data segments, the number of heartbeats of the user during the first period of time.

In some implementations, the acceleration data can include a plurality of acceleration signals, where each of the acceleration signals represents acceleration in a different respective spatial dimension.

In some implementations, determining the second physiological data can include: segmenting the acceleration data into a plurality of acceleration data segments, where each of the acceleration data segments represents a movement of the user during a different respective epoch of the first period of time; determining, for each of the epochs, a variation of the acceleration data during that epoch; and determining, based on the variation of the acceleration data during each of the epochs, the second physiological data.

In some implementations, each of the epochs can correspond to a different respective time window during the first period of time.

In some implementations, each of the time window can be a 15 second window.

In some implementations, determining the second physiological data can include: determining, for each of the epochs, a mean amplitude deviation of the acceleration data during that epoch; and summing the mean amplitude deviations of the acceleration data during each of the epochs.

In some implementations, determining the second physiological data can include determining a log of the sum of the mean amplitude deviations of the acceleration data during each of the epochs.

In some implementations, determining the second physiological data can include determining the second physiological data based on a regression model having the log of the sum of the mean amplitude deviations of the acceleration data as an input.

In some implementations, determining the biomarker can include determining a ratio of the first physiological data and the second physiological data.

In some implementations, causing the biomarker to be presented to the user can include: determining a plurality of numerical ranges, where each of the numerical ranges corresponds to a different respective health rank; determining that the biomarker is within a first numerical range of the plurality of numerical ranges, where the first numerical range corresponds to a first health rank; and causing the first health rank to be presented to the user.

In some implementations, causing the biomarker to be presented to the user can include presenting, using the display device, a graphical user interface to the user, where the graphical user interface includes a graphical display element presenting the biomarker.

In some implementations, causing the biomarker to be presented to the user can include presenting, using the audio speaker, an audio notification to the user, where the audio notification indicates the biomarker.

In some implementations, the one or more computer processors are can be configured to: determine, based on the biomarker, one or more recommendations for improving a health of the user, and cause the one or more recommendations to be presented to the user.

In some implementations, the one or more computer processors can be further configured to instruct, using at least one of a display screen or an audio speaker, the user to walk during the first period of time.

In some implementations, the one or more computer processors can be further configured to: cause the sensor apparatus to obtain the cardiac data and the acceleration data continuously during a second period of time, where the first period of time is a subset of the second period of time; and select the first period of time based on at least one of the cardiac data or the acceleration data.

In some implementations, the one or more computer processors can be further configured to cause the biomarker to be presented to at least one of the user or an additional user.

In another aspect, a method includes: causing, using an electronic device, one or more cardiac sensors of a sensor apparatus to obtain cardiac data regarding a user during a first period of time; causing, using the electronic device, one or more acceleration sensors of the sensor apparatus to obtain acceleration data regarding the user during the first period of time; determining, by the electronic device and based on the cardiac data, first physiological data representing a cardiac activity of the user during the first period of time; determining, by the electronic device and based on the acceleration data, second physiological data representing a variation of the acceleration data during the period of time; determining, by the electronic device and based on the first physiological data and the second physiological data, a biomarker representing a health of the user; and storing, by the electronic device, a data structure presenting the biomarker.

Other embodiments of this aspect include corresponding computer systems, apparatus, and computer programs recorded on one or more computer storage devices, each configured to perform the actions or operations described herein. A system of one or more computers can be configured to perform particular actions by virtue of having software, firmware, hardware, or a combination of them installed on the system that in operation causes or cause the system to perform the actions. One or more computer programs can be configured to perform particular actions by virtue of including instructions that, when executed by a data processing apparatus, cause the apparatus to perform the actions.

The details of one or more embodiments of the subject matter of this specification are set forth in the accompanying drawings and the description below. Other features, aspects, and advantages of the subject matter will become apparent from the description, the drawings, and the claims.

BRIEF DESCRIPTION OF THE DRAWINGS

FIG. 1 is a diagram of an example medical monitoring system.

FIG. 2 is a diagram of an example operation of a medical monitoring system.

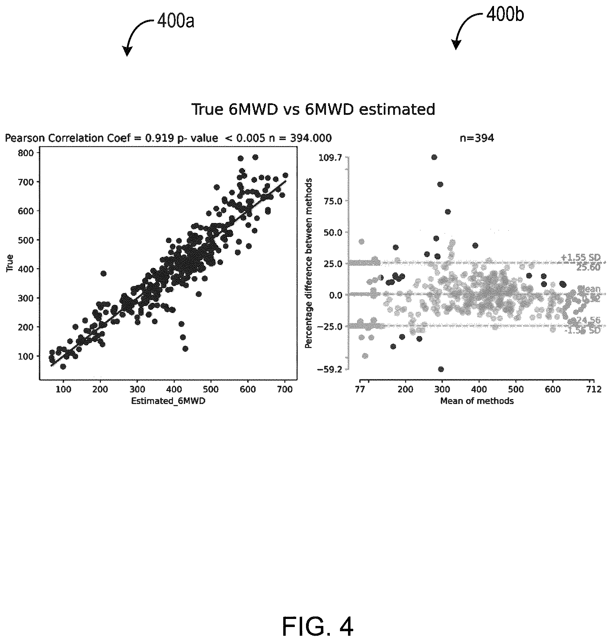

FIGS. 3-7 shows plots representing the results of experimental studies that were performed to evaluate the performance of an example motion metric.

FIG. 8 is a flow chart diagram of an example process for monitoring a user's health.

FIG. 9 is a diagram of an example computer system.

Like reference numbers and designations in the various drawings indicate like elements.

DETAILED DESCRIPTION

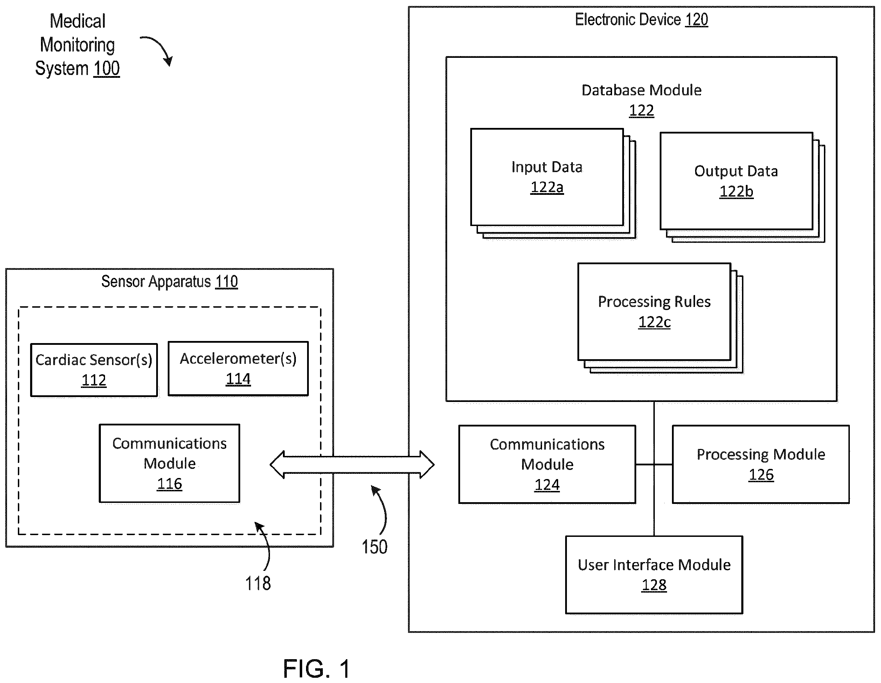

FIG. 1 shows an example medical monitoring system 100 for automatically monitoring the health of a user. In general, the user can be any entity or individual for which medical information is to be obtained (e.g., for personal use and/or to aid in a medical treatment or study).

The medical monitoring system 100 includes a sensor apparatus 110 and an electronic device 120 that are communicatively coupled to one another (e.g., via one or more wired or wireless communications links 150). In general, the medical monitoring system 100 obtains sensor data regarding a user using the sensor apparatus 110, and processes the sensor data using the electronic device 120 to determine one or more biomarkers representing the user's medical condition.

The sensor apparatus 110 includes one or more sensors configured to obtain measurements regarding a physiology of the user, a behavior of the user, and/or any other characteristics of the user.

For example, as shown in FIG. 1, the sensor apparatus 110 can include one or more cardiac sensors 112 configured to obtain sensor data representing the cardiac activity of a user. In some implementations, the cardiac sensor(s) 112 can include or more electrocardiogram (ECG) sensors. In some implementations, the cardiac sensors 112 can obtain sensor data at 250 Hz (or approximately 250 Hz).

Further, the sensor apparatus 110 can include one or more accelerometers 114 configured to obtain sensor data representing a movement or motion of the user in one or more directions. For example, at least some of the accelerometers 114 can be tri-axis accelerometers that are configured to measure acceleration in three directions (e.g., the x-direction, the y-direction, and the z-direction on a Cartesian coordinate system). In some implementations, the accelerometers 114 can obtain sensor data at 32 Hz (or approximately 32 Hz).

Further, the sensor apparatus 110 includes a communications module 116 configured to transmit data and/or receive data from the electronic device 120. As an example, the communications module 116 can include one or more receivers, transmitters, and/or transceivers. In some implementations, the communications module 116 can communicate with the electronic device 120 via one or more wireless links (e.g., serial links, Ethernet links, etc.) and/or wireless links (e.g., Wi-Fi links, Bluetooth links, etc.).

Further, the sensor apparatus 110 is configured to be worn by a user. For example, as shown in FIG. 1, the sensor apparatus 110 can include a substrate 118 that is configured to be affixed to the user's body (e.g., via an adhesive). Further, the cardiac sensors 112, accelerometer 114, and communications module 116 can be attached to the substrate, such that they are secured to the user's body. In some implementations, the at least a portion of the substrate 118 can be composed of compliant materials such as silicone, rubber, elastomers, and/or any other flexible material. In some implementations, the at least a portion of the compliant substrate 118 can be composed of rigid materials such as rigid metals, rigid plastics, etc.

In general, the electronic device 120 is configured to receive sensor data obtained by the sensor apparatus 110, and process the sensor data to determine one or more biomarkers representing the user's medical condition. Further, the electronic device is configured to present information regarding the biomarkers and any other information to the user and/or another user (e.g., a health care provider).

In general, the electronic device 120 can include any number of devices that are configured to receive, process, and transmit data. Examples of the electronic device 120 include client computing devices (e.g., desktop computers or notebook computers), server computing devices (e.g., server computers or cloud computing systems), mobile computing devices (e.g., cellular phones, smartphones, tablets, personal data assistants, notebook computers with networking capability), wearable computing devices (e.g., smart phones or headsets), and other computing devices capable of receiving, processing, and transmitting data. In some implementations, the electronic device 120 can include computing devices that operate using one or more operating systems (e.g., Microsoft Windows, Apple macOS, Linux, Unix, Google Android, and Apple iOS, among others) and one or more architectures (e.g., x86, PowerPC, and ARM, among others).

In FIG. 1, the electronic device 120 is illustrated as a single component. However, in practice, the electronic device 120 can be implemented on one or more computing devices (e.g., each computing device including at least one processor such as a microprocessor or microcontroller). As an example, the electronic device 120 can be a single computing device, such as a single smartphone. As another example, the electronic device 120 can include multiple computing devices that are connected via a network (e.g., the Internet, local area network etc.), and the components of the electronic device 120 can be maintained and operated on some or all of the computing devices. For instance, electronic device 120 can include several computing devices, and the components of the electronic device 120 can be distributed on one or more of these computing devices.

As shown in FIG. 1, the electronic device 120 includes a database module 122, a communications module 124, a processing module 126, and a user interface module 128. The operation modules can be provided as one or more computer executable software modules, hardware modules, or a combination thereof. For example, one or more of the operation modules can be implemented as blocks of software code with instructions that cause one or more processors to execute operations described herein. In addition, or alternatively, one or more of the operations modules can be implemented in electronic circuitry such as, e.g., programmable logic circuits, field programmable logic arrays (FPGA), or application specific integrated circuits (ASIC).

The communications module 124 is configured to transmit data and/or receive data from the sensor apparatus 110. As an example, the communications module 124 can include one or more receivers, transmitters, and/or transceivers. In some implementations, the communications module 124 can communicate with the sensor apparatus 110 (e.g., via the communication module 116) via one or more wireless links (e.g., serial links, Ethernet links, etc.) and/or wireless links (e.g., Wi-Fi links, Bluetooth links, etc.).

The database module 122 maintains information related to the operation of the medical monitoring system 100.

As an example, the database module 122 can store input data 122a that is used as an input for determining one or more biomarkers representing a health of a user. For instance, the input data 122a can include at least some of the sensor data generated by the sensor apparatus 110 (e.g., cardiac data, acceleration data, etc.).

As another example, the database module 122 can store output data 122b generated by electronic device 120. As an example, the output data 122b can include one or more metrics or biomarkers generated by the electronic device 120 based on the input data 122a.

Further, the database module 122 can store processing rules 122c specifying how data in the database module 122 can be processed to perform the operations described herein.

As an example, the processing rules 122c can include one or more rules that specify how the input data 122a is formatted, parsed, and processed to determine one or more corresponding metrics or biomarkers regarding a user.

As another example, the processing rules 122c can include one or more rules that specify the conditions in which data is presented to a user (e.g., using the user interface module 128), and the manner in which the data is presented.

As another example, the processing rules 122c can include one or more rules that specify the manner in which data is stored for future retrieval and/or processing (e.g., using the database module 122).

As another example, the processing rules 122c can include or more rules to generate and/or modify the input data 112a, such that has a particular pre-determined format. This can be beneficial, for example, in improving the capability of the electronic device 120 with multiple different types of sensors (e.g., each of which may output data having a different respective format). As an example, the processing rules 112c can specify that sensor data be processed such that each particular sensor data type, column names and sample frequency resolution are converted into a common data format. Using a common data format enables data obtained from multiple sensors and/or sensor types to be processed in consistent manner. Further, the processing rules 112c can include rules for correcting data errors, removing noise, handling (e.g., removing) data outliers, and determining that the data is suitable for further analysis. Further, the processing rules 112 can include rules for generating a log of the data cleaning activities, including the actions taken and any issues encountered. This log can be used to generate a report that summarizes the data cleaning process and its outcomes.

Example data processing techniques are described in further detail below.

The processing module 126 processes data stored or otherwise accessible to the electronic device 120. For instance, the processing module 126 can be used to execute one or more of the operations described herein (e.g., by executing the processing rules 122c with respect to the input data 112a in order to generate the output data 122b).

The user interface module 128 is configured to present information to a user and/or to receive inputs from a user. As an example, the user interface module 128 can include one more display devices (e.g., display screens, touch screens, etc.) that are configured to present a user interface (e.g., graphical user interface, GUI) that enables users to interact with the electronic device 120 and/or the sensor apparatus 110. Example interactions include viewing data, transmitting data from one component to another, and/or issuing commands to the electronic device 120 and/or sensor apparatus 110. Commands can include, for example, any user instruction to one or more of the electronic device 120 and/or sensor apparatus 110 to perform particular operations or tasks. In some implementations, the user interface module can also present information to a user aurally (e.g., using one or more speakers) and/or via haptic feedback (e.g., using one more haptic generators, such as a vibration generation).

In some implementations, a software application can be used to facilitate performance of the tasks described herein. As an example, an application can be installed on the electronic device 120. Further, a user can interact with the application to input data and/or commands to the electronic device 120, and review data generated by the call electronic device 120.

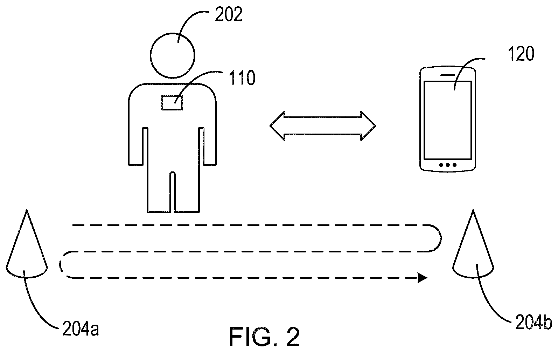

FIG. 2 illustrates an example implementation and operation of the medical monitoring system 100.

In this example, the sensor apparatus 110 is worn by a user 202 (e.g., affixed to the user's skin, such as on the user's chest). Further, the user 202 continuously walks for period of time. For example, the user 202 can repeatedly walk from point 204a and point 204b, and back again, from the beginning of the period of time until the end of the period of time. As the user 202 is walking, the sensor apparatus 110 obtains sensor data regarding the user 202 (e.g., cardiac data, acceleration data, etc.), and transmits at least some of the sensor data to the electronic device 120 for further processing.

In at least some implementations, the user 202 can walk for a period of time spanning six minutes. This can be beneficial, for example, in causing the user 202 to expend a sufficient amount of physical effort, such that the health of the user 202 can be accurately and reliably ascertained using sensor data obtained during that period of time. Nevertheless, in some implementations, other periods of time can be used (e.g., one minute, two minutes, three minutes, etc., or any other period of time).

Further, in at least some implementations, the distance between point 204a and point 204b can be approximately 30 feet. This can be beneficial, for example, as it provides enough space for the user 202 to walk continuously over a time interval before having the turnaround. Further, the space is compact enough that it can be readily found in many common environments (e.g., the user's home, office, etc.). Nevertheless, in some implementations, other distances can be used (e.g., 20 feet, 40 feet, 50 feet, etc., or any other distance).

The electronic device 120 receives the sensor data, and processes the sensor data to determine a biomarker that represents the user's physical health. For example, the electronic device 120 can determine a biomarker that represents the user's functional capacity (e.g., the user's ability to function in a variety of circumstances, such as the user's ability to pertain physical activities) and/or cardiopulmonary condition (e.g., the health of the user's heart, lungs, circulatory system, etc.). Further, the electronic device 120 can present the biomarker to the user 202 or another user (e.g., a health care provider) to facilitate treatment of the user 202 and/or monitoring of the user's health over time.

In some implementations, the medical monitoring system 100 can instruct the user regarding how to conduct a test using the medical monitoring system 100. For example, the medical monitoring system 100 can instruct the user (e.g., using a display screen, audio speaker, etc.) to place the sensor apparatus 110 on their body, select two points that are a particular distance apart from one another (e.g., 30 feet), and walk continuously between those two points during a particular period of time (e.g., 6 minutes). As described above, the medical monitoring system 100 can determine the biomarker based on the sensor data.

In some implementations, the medical monitoring system 100 can continuously obtain sensor data regarding the user (e.g., during the course of the day), and automatically select a period of time for which to determine the biomarker. For example, the medical monitoring system 100 can determine, based on the sensor data, that the user has been continuously walking during a particular period of time (e.g., a continuous six-minute window). The medical monitoring system 100 can select sensor data from that period of time, and determine the biomarker based on the selected sensor data. This can be beneficial, for example, as it allows the medical monitoring system 100 to unobtrusively monitor the user's health over time (e.g., without requiring that the user manually initiate a test and/or without requiring that the user perform a prescribed physical activity).

In some implementations, the biomarker can be determined, at least in part, by determining a ratio between (i) a number of heartbeats by the user over the period of time (e.g., 6 minutes), and (ii) a motion metric representing a variation in the movements of the user over that period of time. As an example, the biomarker X can be determined using the relationship:

$$X = \frac{(heartbeats)}{(motion\ metric)} \quad (\text{Eq. 1}),$$

Where *heartbeats* is the number of heartbeats by the user over the period of time, and *motion metric* represents a variation in the movements of the user over that period of time.

In some implementations, the number of heartbeats can be determined by segmenting the cardiac data (e.g., ECG data) into several segments, where each segment corresponds to a different respective single heartbeat. For example, the cardiac data can be segmented by identifying features of the cardiac data (e.g., peaks, valleys, etc.) that are indicative of a heartbeat). Further, the number of segments can be counted to determine the total number of heartbeats.

In general, the motion metric can be determined based on a mean amplification deviation (MAD) of the acceleration data (e.g., as obtained by the accelerometer(s) 114) over a particular period of time. Example techniques for determining the motion metric are described in further detail below.

Further, the value of the biomarker can represent the relative health of the user. For example, over a particular range of values of the biomarker, a lower value can indicate that the user health is relatively high, whereas a higher value for the biomarker can indicate that the user health is relatively low.

As an illustrative example, during a period of time, a first user and a second user walk in a similar manner. Thus, the motion metric representing the variation in the movements of the users are the same. However, in this example, the first user's heartbeats during the period of time is greater than that of the second user, which may indicate that the first user's functional ability and/or cardiopulmonary health is worse than that of the second user. This difference reflected in the biomarkers of the two users, in which the value of the first user's biomarker is greater than that of the second user.

As another example, during a period of time, a first user and a second user have the same number of heartbeats. However, the first user's physical activity during the period of time was greater than that of the second user (e.g., reflected by a greater degree of variation in the movements of the first user, compared to that of the movements of the second user). This may indicate that the first user's functional ability and/or cardiopulmonary health is better than that of the second user. This difference is reflected in the biomarkers of the two users, in which the value of the first user's biomarker is less than that of the second user.

In some implementations, the medical monitoring system 100 can present the biomarker to the user and/or another user (e.g., a health care provider). For example, the medical monitoring system 100 can present the biomarker to the user and/or another user using the user interface module 128 (e.g., graphically, aurally, haptically, etc.). As another example, the medical monitoring system 100 can transmit data representing the biomarker to another device (e.g., a remote computer system, cloud computing platform, etc.), which in turn causes the biomarker to be presented to the user and/or another user (e.g., graphically, aurally, haptically, etc.).

In some implementations, the medical monitoring system 100 can present value of the biomarker (e.g., the calculated ratio between (i) a number of heartbeats by the user over the period of time, and (ii) a motion metric representing a variation in the movements of the user over that period of time). For example, the value of the biomarker can be presented as a graphical display element on a GUI (e.g., as presented on a display device) and/or as an audio notification (e.g., as presented using an audio speaker).

In some implementations, the medical monitoring system 100 can determine a descriptive health category of the user based on the value of the biomarker. For example, medical monitoring system 100 can access several health categories (e.g., "good," "fair," "poor," etc.), each associated with a different respective range of values. The medical monitoring system 100 can determine that the value of the biomarker falls within a particular one of the ranges, and determine that the health of the user corresponds to the category associated with that range. This can be beneficial, for example, as a descriptive health category may be easier for a user to understand, compared to the specific value of biomarker.

In some implementations, the medical monitoring system 100 can also determine additional information regarding the physiology of the user. For example, based on the cardiac data, the medical monitoring system 100 can determine cardiac metrics such as the user's resting and peak heart rates, and heart rate recovery after the walk. As another example, based on the acceleration data, the medical monitoring system 100 can determine gait metrics regarding the user's walking patterns. This additional information also can be presented to the user and/or another user (e.g., graphically, aurally, haptically, etc.).

In some implementations, the medical monitoring system 100 can be used to facilitate treatment of user 202, thereby improving the user's health. For example, the medical monitoring system 100 can generate a biomarker representing the user's health, and provide the biomarker to the user 202 or another user (e.g., a health care provider). Based on this information, the user 202 or other user can take active steps to improve the user's health, such as conducting further tests to diagnose the user's medical condition, changing the user's behavior, lifestyle, and/or diet, or any other action.

In some implementations, medical monitoring system 100 can determine one or more recommendations for improving a health of the user based on the biomarker, and cause the recommendations to be presented to the user.

In some implementations, the medical monitoring system 100 can also provide instructions to the user 202 regarding how to operate the medical monitoring system 100. For example, the medical monitoring system 100 (e.g., using the electronic device 120) can provide instructions to the user 202 to affix the sensor apparatus 110 to a particular location on the user's body (e.g., the user's chest), and to walk back and forth between two points for a particular length of time (e.g., six minutes). Further, upon completion of the user's walk, the medical monitoring system 100 can provide feedback to the user 202 regarding the sensor data that was collected and the biomarker that was generated based on the sensor data. This can be beneficial, for example, in aiding the user 202 to conduct a test of their physical health, even if the user 202 does not have any prior experience with conducting the test. In some implementations, the instructions can be provided by the medical monitoring system 100 graphically (e.g., using one or more display screens and/or aurally (e.g., using one or more audio speakers).

*Examples Techniques Calculating a Motion Metric*

Example techniques for calculating the motion metric are described in further detail below.

In some implementations, the motion metric can represent a mean amplification deviation (MAD) of the acceleration data (e.g., as obtained by the accelerometer(s) 114) over a particular period of time. As an example, in at least some implementations, the MAD can be the mean distance of data points about the mean of three-axes of the accelerometer data over a period of time, according to the relationship:

$$MAD = \frac{1}{n} * \sum \left\| r_i - \underline{r} \right\| \qquad \text{(Eq. 2)},$$

where:

$$r_i = (x_i^2 + y_i^2 + z_i^2)^{1/2} \qquad \text{(Eq. 3)},$$

$$\underline{r} = \frac{1}{n} \sum_{i=0}^{n} |r_i| \qquad \text{(Eq. 4)},$$

$n$ is the number of samples in each window, $x, y,$ and $z$ are the three-axes of the accelerometer data (e.g., according to a Cartesian coordinate system).

In at least some implementations, MAD can be used as an objective measure of a user's total activity during a period of time (e.g., during a six-minute walk).

In general, MAD can be used to estimate the distance that a user walked during a particular period of time. For example, in a traditional "six-minute walk test," a user walks continuously during a six minute period of time. Upon the completion of the period of time, an observer (e.g., a health care provider) measures the distance that the user has walked, and uses the measured distance as a biomarker representing the user's health. The measured distance may be referred to as the six-minute walk distance (6MWD).

MAD can be used to approximate the 6MWD, without requiring that a user manually measure the distance that a user has walked. In particular, MAD can be used to calculate the estimated 6MWD ($6MWD_{estimated}$) according to the relationship:

$$MAD_{epoch} = \frac{1}{n}\sum_{i=0}^{n}|r_i - \underline{r}| \qquad (\text{Eq. 5}),$$

Where:

$$r_i = \sqrt{(x_i^2 + y_i^2 + z_i^2)} \qquad (\text{Eq. 6}),$$

$$\underline{r} = \frac{1}{n}\sum_{i=0}^{n}|r_i| \qquad (\text{Eq. 7}),$$

$$MAD_{SUM} = \sum_{j=1}^{24} MAD_{epoch}^{j} \qquad (\text{Eq. 8}).$$

Here, $n$ is the total number of samples in each epoch, $i$ is the index of each sample in the epoch, and $x, y, z$ are the raw values of the three-axes from the accelerometer data.

Raw acceleration data is collected while the user walks for six minutes. Further, the raw acceleration data is segmented into a number of epochs, each spanning a particular period of time. For example, the raw acceleration data can be segmented in 24 epochs, each spanning 15 seconds. This may be particularly suitable for use in measuring the user's physical exertion while walking over a distance of approximately 30 feet (e.g., the user is less likely to turn around multiple times during a particular epoch, which may skew or distort the results). Nevertheless, in practice, the raw acceleration data can be segmented into a different number of epochs, each spanning a different length of time.

Further, the MAD is calculated for each of the epochs (e.g., using Equations 4-6 above).

Further, the sum of $MAD_{epoch}$ is calculated over all the epochs (e.g., using Equation 7 above), which results in a feature $MAD_{SUM}$ for the six minutes of data for each walk.

*Example Experimental Studies*

As described in further detail below, experimental studies were performed to demonstrate the accuracy, repeatability, reliability, and hardware independence of a MAD-based biomarker for functional capacity

Estimation of Sensor Derived Six-Minute Walk Test Distance ($MWD_{estimated}$ Metric):

$MAD_{SUM}$ was calculated for each of several walks by a group of users. When $MAD_{SUM}$ was plotted for the different walks and compared with the actual distance that was walked by a user (e.g., as measured by an in-clinic observer), the relationship followed a quadratic curve.

Further, a logarithm of $MAD_{SUM}$ was calculated (e.g., log ($MAD_{sum}$)) to convert this quadratic relationship to a linear relationship.

Further, a regression model was developed to predict the 6MWD when we are given the $log(MAD_{SUM})$ for a walk derived from the accelerometer data:

$$6MWD_{estimated} = m * \log(MAD_{SUM}) + c \qquad (\text{Eq. 7}).$$

Using data from both long and short in-clinic walks, the slope m and intercept c for the above equation were derived as

$$m = 226.277,$$

and

$$c = 89.15,$$

with a coefficient of determination of 0.845 and a mean square error of 3194.260. A coefficient of determine of approximately 0.85 indicates that almost 15% variability in the data is not accounted for by the model that has been fitted.

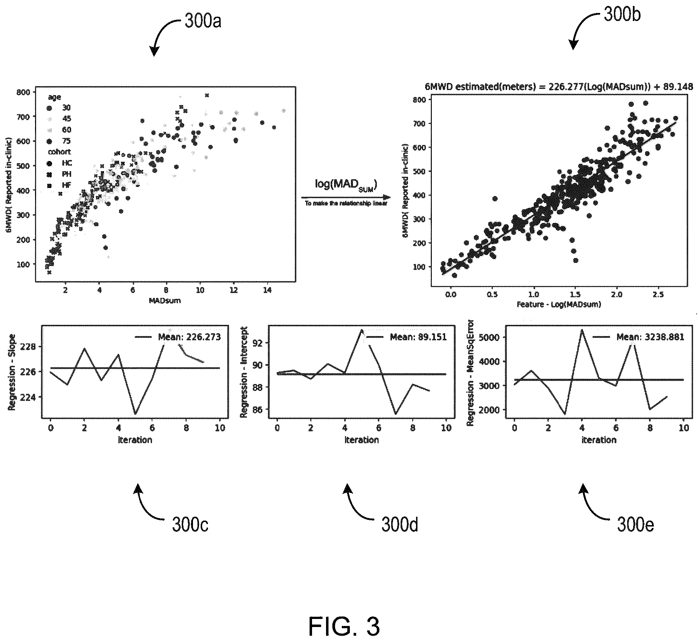

The results of the regression model development are shown in FIG. 3. In particular, FIG. 3 shows a scatter plot between $MAD_{SUM}$ and 6MWD (e.g., as measured by an in-clinic observer) (plot 300a), and scatter plot between log($MAD_{SUM}$) and 6MWD (plot 300b).

To further evaluate the above regression model, a k-fold cross validation was performed with k = 10. FIG. 3 shows the slopes (plot 300c), the intercepts (plot 300d), and the MSEs (plot 300e) of the different iterations when evaluated using the k-fold cross validation. The average mean square error from k-fold cross-validation was 3238.9 units (plot 300e) and the mean square error from the regression fit was 3194.26 units. Further, the average slope from k-fold cross-validation was 226.27 (plot 300c) and the slope from the regression fit was 226.27. Further, the average intercept from k-fold cross-validation was 89.15 (plot 300d) and the intercept from the regression fit was 89.15. The consistency between the average mean square error obtained from k-fold cross validation and the mean square error from the regression fit indicates the stability and reliability of the model. Furthermore, the closeness in the values of the slope and intercept derived from both methods underscores the robustness of the model parameters. Such consistency suggests that the model's performance is not overly sensitive to the particular data partition used during validation and it is expected to exhibit similar performance on new unseen data.

To assess the agreement between the predicted values obtained post regression and the true values, two evaluation techniques were performed.

First, a Pearson correlation coefficient was calculated to measure the linear relationship between the two sets of values. As shown in FIG. 3, a scatter plot 400a shows a high degree of correlation between the $6MWD_{estimated}$ and 6MWD ("True") evaluated on n = 394 walks. The obtained correlation coefficient was 0.91 indicating a strong linear association.

Second, a Bland-Altman plot was constructed to visualize the agreement between the two measurements by plotting the percentage difference against the mean of the predicted and the true values (plot 400b). The mean difference observed was 0.52% and the limits of agreements range from +25% to -25%, providing an insight into the consistency and potential discrepancies between the predicted values ($6MW D_{estimated}$) and the actual values (6MWD, or "True").

Repeatability Across Shorter Walkway Course Lengths:

Further, experiments were conducted to determine whether the six-minute walk test (6MWT) can be adapted to an out-of-clinic setting using sensor technology for remote 6MWT assessment.

Although the American Thoracic Society (ATS) 6MWT guidelines recommend a long (e.g., 100 feet), flat, straight, and unobstructed hallway for the test to be performed, this specified testing environment is often not well-suited for a home setting, as a hallway of this length is uncommon in a standard home. Therefore, the independence of the $6MW D_{estimated}$ metric from the course length was examined. In these experiments, a shorter 30 foot course length was selected, and data was collected during a 6MWT in-clinic (e.g., including measurements of the walked distance by a clinical observer).

First, a comparison was made between (i) the Pearson correlation coefficient between a 90 foot walkway and a shorter 30 foot walkway for the traditional 6MWD metric reported by the clinician at site and (ii) the correlation coefficient between the two walkway courses for the $6MW D_{estimated}$ derived from the MAD of the acceleration data.

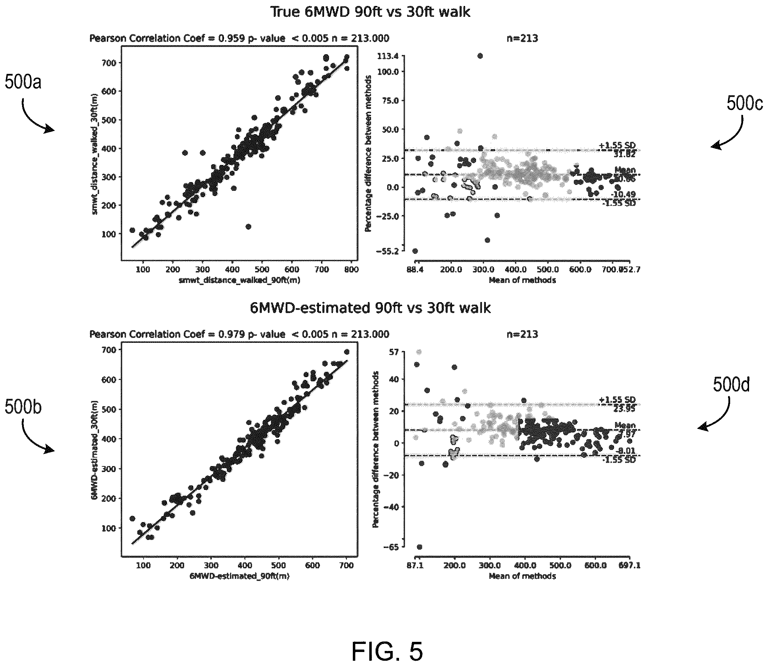

The results of this comparison are shown in FIG. 5. In particular, FIG. 5 shows a comparison between the traditional 6MWD metric and the $6WMD_{estimated}$ metric over two different walkway lengths, including (i) the Pearson correlation coefficient (plots 500a and 500b), and (ii) Bland Altman plots visualizing the agreement and bias (plots 500c and 500d).

As shown in FIG. 5, the Pearson correlation coefficient for the traditional 6MWD metric is 0.96, and the Pearson correlation coefficient for the $6MW D_{estimated}$ metric is 0.98. This highlights the consistency of the $6MW D_{estimated}$ metric across varying course lengths.

Further, Bland-Altman plots were constructed for both the traditional and the estimated 6WMD to visualize the agreement across the two walkway lengths (90 feet and 30 feet). In the case of the traditional 6MWD metric, the average bias was 10.66%, with limits of agreement ranging from 32% to -11% (plot 500c). In comparison, the $6MW D_{estimated}$ metric demonstrated a tighter agreement with an average bias of 8% and limits of agreement ranging from 24% to -8% (plot 500d). These results demonstrate the robustness of the $6MWD_{estimated}$ metric, and emphasizes its utility as an accurate and reliable tool for functional assessment across diverse walkway lengths.

Repeatability Across Visits In-Clinic Under Supervision:

To appraise the repeatability of both the traditional 6MWD metric and the $6MW\ D_{estimated}$ metric over time, measurements from two separate visits ("Baseline" and "Week 8") were analyzed.

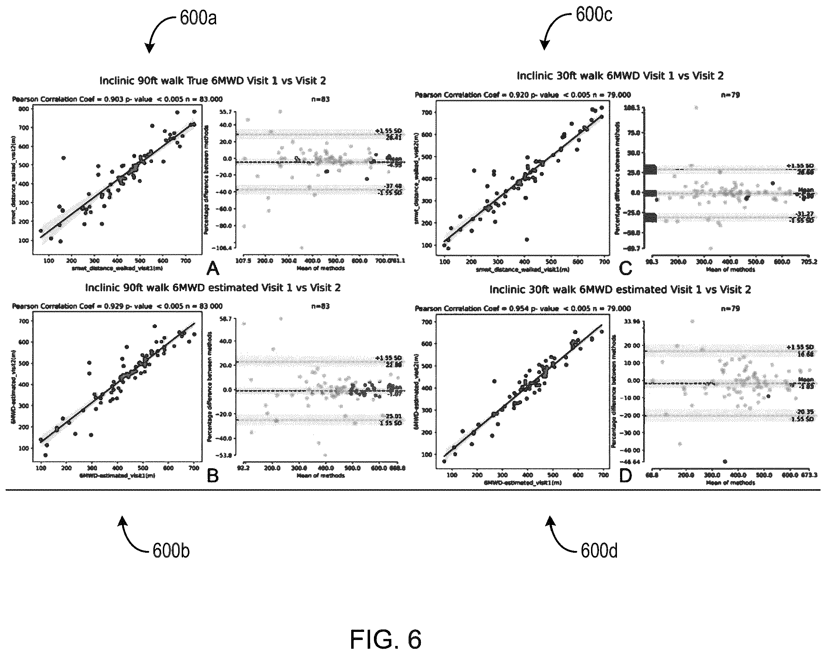

The results of this study are shown in FIG. 6. In particular, FIG. 6 shows Pearson correlation coefficients and Bland-Altman plots for standard course (plots 600a and 600b). Further, FIG. 6 shows corresponding plots for the shorter course length (plots 600c and 600d). The analyses underscore the superior repeatability of the $6MW\ D_{estimated}$ metric across visits and walkway lengths.

In particular, for a standard walkway course length of 90 feet (plot 600a, left), the 6MWD metric exhibited a Pearson correlation coefficient of 0.903 between the visits and the Bland-Altman plots (plot 500a, right) for the same revealed an average bias of -4.53%, with limits of agreement spanning from 28.4% to -37.48%. In contrast, the $6MW\ D_{estimated}$ metric on the standard course demonstrated superior repeatability with a correlation coefficient of 0.93 (plot 600b, left) and a significantly lower average bias of - 4.53% from the Bland-Altman with limits of agreement ranging from 22.8% to -25% (plot 600b, right).

When assessed on the shorter course length, a similar trend persisted. The traditional 6MWD metric generated a Pearson coefficient of 0.92 (plot 600c, left), and Bland-Altman bias of -1.28% (plot 600c, right), with limits of agreement between 28.69% to -31.27%. In contrast, the $6MW\ D_{estimated}$ metric again emerged more repeatable with respective figures of Pearson coefficient of 0.954 (plot 600d, left), and Bland-Altman bias of -1.83% (plot 600d, right), with limits of agreement between 16.68% to -20.35%. Collectively, these findings accentuate the augmented repeatability of he $6MW\ D_{estimated}$ metric over time, irrespective of the course length, endorsing its utility in consistent functional assessment.

Repeatability of $6MWD_{estmated}$ Metric Across Visits At-Home Without Supervision:

Expanding the evaluation to a home-based context, the repeatability of the $6MW\ D_{estimated}$ metric was assessed over two distinct visits.

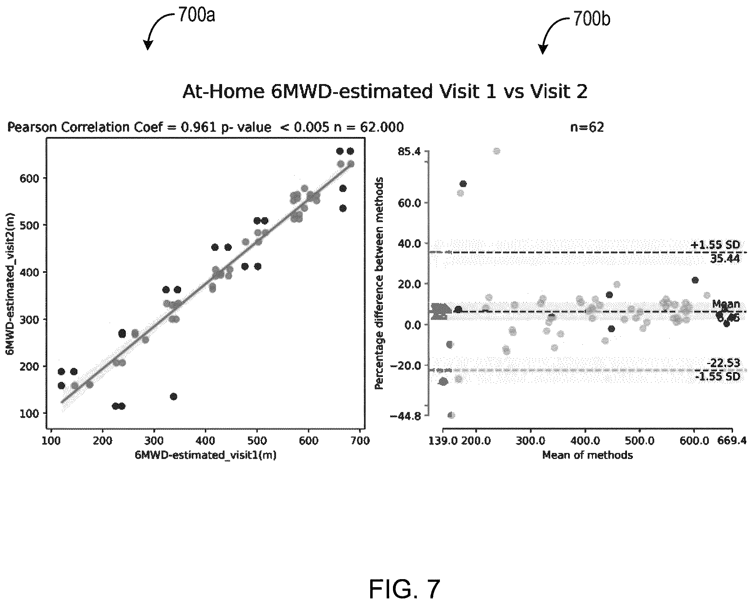

The results of this evaluation are shown in FIG. 7. In particular, FIG. 7 shows
(Left) Pearson correlation coefficient indicating the linear consistency between visits. (Right) Bland-Altman plot visualizing the agreement and bias across the two home-based measurements. Results emphasize the metric's consistent performance in the home setting.

In this more informal and patient-convenient setting, the metric continued to show promising consistency. The Pearson correlation coefficient between visits was 0.961, suggesting a strong degree of repeatability (e.g., linear consistency) between visits (FIG. 7, plot 700a). Further insights were drawn from the Bland-Altman plots which depicted an average bias of 6.45%, with limits of agreement delineated between 35% and -22.5% (FIG. 7, plot 700b). The robust repeatability observed, even in the variable environment of the home, underscores the adaptability and potential of he $6MW\ D_{estimated}$ metric for decentralized functional assessments.

*Example Processes*

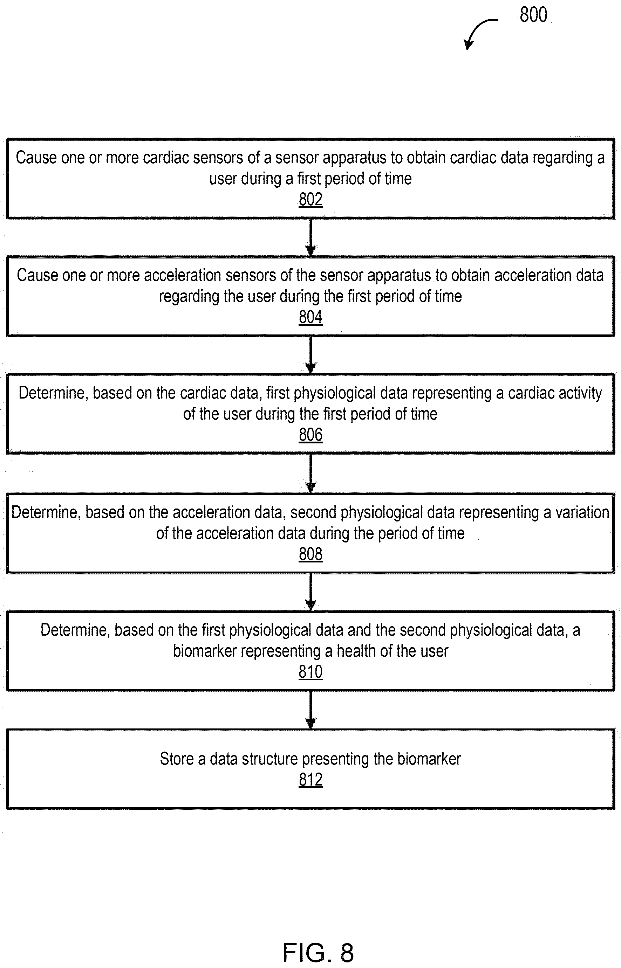

FIG. 8 shows an example process 800 for monitoring a user's medical condition using physiological sensors. In some implementations, the process 800 can be performed by the medical monitoring system 100 described in this disclosure.

In the process 800, a system causes one or more cardiac sensors of a sensor apparatus to obtain cardiac data regarding a user during a first period of time (802). In some implementations, the first period of time can be six minutes. In some implementations, the one or more cardiac sensors can include an electrocardiogram (ECG) sensor.

Further, the system causes one or more acceleration sensors of the sensor apparatus to obtain acceleration data regarding the user during the first period of time (804). In some implementations, the acceleration data can include a plurality of acceleration signals, where each of the acceleration signals represents acceleration in a different respective spatial dimension.

In some implementations, the sensor apparatus can be configured to be attached to the user's chest.

In some implementations, the system can instruct, using at least one of a display screen or an audio speaker, the user to walk during the first period of time.

In some implementations, the system can cause the sensor apparatus to obtain the cardiac data and the acceleration data continuously during a second period of time, where the first period of time is a subset of the second period of time. Further, the system can select the first period of time based on at least one of the cardiac data or the acceleration data.

Further, the system determines, based on the cardiac data, first physiological data representing a cardiac activity of the user during the first period of time (806). In some implementations, the first physiological data can represent a number of heartbeats of the user during the first period of time.

In some implementations, determining the first physiological data can include segmenting the cardiac data into a plurality of cardiac data segments, where each of the cardiac data segments represents a different respective heartbeat of the user.

In some implementations, determining the first physiological data can include determining, based on the cardiac data segments, the number of heartbeats of the user during the first period of time.

Further, the system determines, based on the acceleration data, second physiological data representing a variation of the acceleration data during the period of time (808).

In some implementations, determining the second physiological data can include: segmenting the acceleration data into a plurality of acceleration data segments, where each of the acceleration data segments represents a movement of the user during a different respective epoch of the first period of time; determining, for each of the epochs, a variation of the acceleration data during that epoch; and determining, based on the variation of the acceleration data during each of the epochs, the second physiological data.

In some implementations, each of the epochs can correspond to a different respective time window during the first period of time.

In some implementations, each of the time window can be a 15 second window.

In some implementations, determining the second physiological data can include: determining, for each of the epochs, a mean amplitude deviation of the acceleration data during that epoch; and summing the mean amplitude deviations of the acceleration data during each of the epochs.

In some implementations, determining the second physiological data can include determining a log of the sum of the mean amplitude deviations of the acceleration data during each of the epochs.

In some implementations, determining the second physiological data can include determining the second physiological data based on a regression model having the log of the sum of the mean amplitude deviations of the acceleration data as an input.

Further, the system determines, based on the first physiological data and the second physiological data, a biomarker representing a health of the user (810). In some implementations, the biomarker can represent a functional capacity of the user. For example, the biomarker can represent a cardiopulmonary condition of the user.

In some implementations, determining the biomarker can include determining a ratio of the first physiological data and the second physiological data.

Further, the system stores a data structure presenting the biomarker (812).

In some implementations, the system can also cause the biomarker to be presented to the user and/or an additional user.

For example, the system can present, using a display device, a graphical user interface to the user, where the graphical user interface includes a graphical display element presenting the biomarker. As another example, the system can present, using an audio speaker, an audio notification to the user, where the audio notification indicates the biomarker.

In some implementations, the system can determine a plurality of numerical ranges, where each of the numerical ranges corresponds to a different respective health rank. Further, the system can determine that the biomarker is within a first numerical range of the plurality of numerical ranges, where the first numerical range corresponds to a first health rank, and cause the first health rank to be presented to the user.

In some implementations, the system can determine, based on the biomarker, one or more recommendations for improving a health of the user, and cause the one or more recommendations to be presented to the user.

*Example Computer Systems*

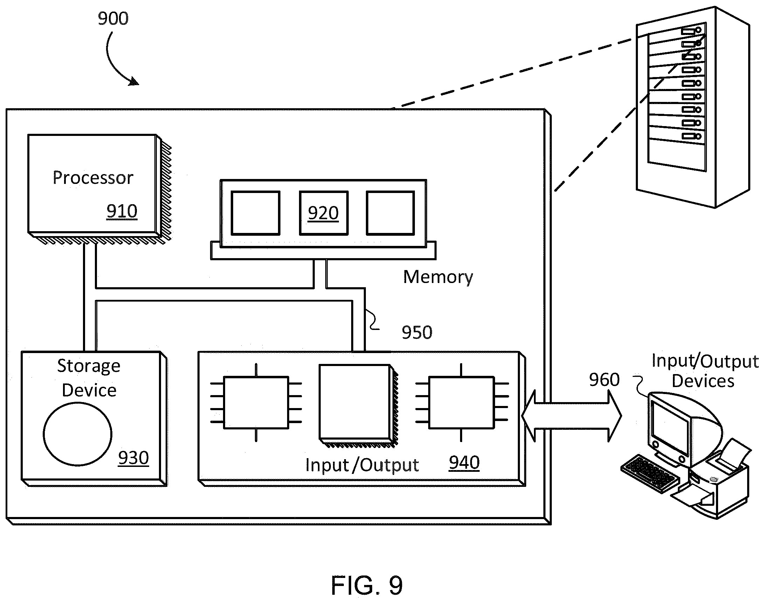

FIG. 9 depicts an example computing system, according to implementations of the present disclosure. The system 900 may be used for any of the operations described with respect to the various implementations discussed herein. The system 900 may include one or more processors 910, a memory 920, one or more storage devices 930, and one or more input/output (I/O) devices 960 controllable through one or more I/O interfaces 940. The various components 910, 920, 930, 940, or 960 may be interconnected through at least one system bus 950, which may enable the transfer of data between the various modules and components of the system 900.

The processor(s) 910 may be configured to process instructions for execution within the system 900. The processor(s) 910 may include single-threaded processor(s), multi-threaded processor(s), or both. The processor(s) 910 may be configured to process instructions stored in the memory 920 or on the storage device(s) 930. The processor(s) 910 may include hardware-based processor(s) each including one or more cores. The processor(s) 910 may include general purpose processor(s), special purpose processor(s), or both.

The memory 920 may store information within the system 900. In some implementations, the memory 920 includes one or more computer-readable media. The memory 920 may include any number of volatile memory units, any number of non-volatile memory units, or both volatile and non-volatile memory units. The memory 920 may include read-only memory, random access memory, or both. In some examples, the memory 920 may be employed as active or physical memory by one or more executing software modules.

The storage device(s) 930 may be configured to provide (e.g., persistent) mass storage for the system 900. In some implementations, the storage device(s) 930 may include one or more computer-readable media. For example, the storage device(s) 930 may include a floppy disk device, a hard disk device, an optical disk device, or a tape device. The storage device(s) 930 may include read-only memory, random access memory, or both. The storage device(s) 930 may include one or more of an internal hard drive, an external hard drive, or a removable drive.

One or both of the memory 920 or the storage device(s) 930 may include one or more computer-readable storage media (CRSM). The CRSM may include one or more of an electronic storage medium, a magnetic storage medium, an optical storage medium, a magneto-optical storage medium, a quantum storage medium, a mechanical computer storage medium, and so forth. The CRSM may provide storage of computer-readable instructions describing data structures, processes, applications, programs, other modules, or other data for the operation of the system 900. In some implementations, the CRSM may include a data store that provides storage of computer-readable instructions or other information in a non-transitory format. The CRSM may be incorporated into the system 900 or may be external with respect to the system 900. The CRSM may include read-only memory, random access memory, or both. One or more CRSM suitable for tangibly embodying computer program instructions and data may include any type of non-volatile memory, including but not limited to: semiconductor memory devices, such as EPROM, EEPROM, and flash memory devices; magnetic disks such as internal hard disks and removable disks; magneto-optical disks; and CD-ROM and DVD-ROM disks. In some examples, the processor(s) 910 and the memory 920 may be supplemented by, or incorporated into, one or more application-specific integrated circuits (ASICs).

The system 900 may include one or more I/O devices 960. The I/O device(s) 960 may include one or more input devices such as a keyboard, a mouse, a pen, a game controller, a touch input device, an audio input device (e.g., a microphone), a gestural input device, a haptic input device, an image or video capture device (e.g., a camera), or other devices. In some examples, the I/O device(s) 960 may also include one or more output devices such as a display, LED(s), an audio output device (e.g., a speaker), a printer, a haptic output device, and so forth. The I/O device(s) 960 may be physically incorporated in one or more computing devices of the system 900, or may be external with respect to one or more computing devices of the system 900.

The system 900 may include one or more I/O interfaces 940 to enable components or modules of the system 900 to control, interface with, or otherwise communicate with the I/O device(s) 960. The I/O interface(s) 940 may enable information to be transferred in or out of the system 900, or between components of the system 900, through serial communication, parallel communication, or other types of communication. For example, the I/O interface(s) 940 may comply with a version of the RS-232 standard for serial ports, or with a version of the IEEE 1284 standard for parallel ports. As another example, the I/O interface(s) 940 may be configured to provide a connection over Universal Serial Bus (USB) or Ethernet. In some examples, the I/O interface(s) 940 may be configured to provide a serial connection that is compliant with a version of the IEEE 1394 standard.

The I/O interface(s) 940 may also include one or more network interfaces that enable communications between computing devices in the system 900, or between the system 900 and other network-connected computing systems. The network interface(s) may include one or more network interface controllers (NICs) or other types of transceiver devices configured to send and receive communications over one or more networks using any network protocol.

Computing devices of the system 900 may communicate with one another, or with other computing devices, using one or more networks. Such networks may include public networks such as the internet, private networks such as an institutional or personal intranet, or any combination of private and public networks. The networks may include any type of wired or wireless network, including but not limited to local area networks (LANs), wide area networks (WANs), wireless WANs (WWANs), wireless LANs (WLANs), mobile communications networks (e.g., 3G, 4G, Edge, etc.), and so forth. In some implementations, the communications between computing devices may be encrypted or otherwise secured. For example, communications may employ one or more public or private cryptographic keys, ciphers, digital certificates, or other credentials supported by a security protocol, such as any version of the Secure Sockets Layer (SSL) or the Transport Layer Security (TLS) protocol.

The system 900 may include any number of computing devices of any type. The computing device(s) may include, but are not limited to: a personal computer, a smartphone, a tablet computer, a wearable computer, an implanted computer, a mobile gaming device, an electronic book reader, an automotive computer, a desktop computer, a laptop computer, a notebook computer, a game console, a home entertainment device, a network computer, a server computer, a mainframe computer, a distributed computing device (e.g., a cloud computing device), a microcomputer, a system on a chip (SoC), a system in a package (SiP), and so forth. Although examples herein may describe computing device(s) as physical device(s), implementations are not so limited. In some examples, a computing device may include one or more of a virtual computing environment, a hypervisor, an emulation, or a virtual machine executing on one or more physical computing devices. In some examples, two or more computing devices may include a cluster, cloud, farm, or other grouping of multiple devices that coordinate operations to provide load balancing, failover support, parallel processing capabilities, shared storage resources, shared networking capabilities, or other aspects.

This specification uses the term "configured" in connection with systems and computer program components. For a system of one or more computers to be configured to perform particular operations or actions means that the system has installed on software, firmware, hardware, or a combination of them that in operation cause the system to perform the operations or actions. For one or more computer programs to be configured to perform particular operations or actions means that the one or more programs include instructions that, when executed by data processing apparatus, cause the apparatus to perform the operations or actions.

Embodiments of the subject matter and the functional operations described in this specification can be implemented in digital electronic circuitry, in tangibly-embodied computer software or firmware, in computer hardware, including the structures disclosed in this specification and their structural equivalents, or in combinations of one or more of them. Embodiments of the subject matter described in this specification can be implemented as one or more computer programs, i.e., one or more modules of computer program instructions encoded on a tangible non transitory storage medium for execution by, or to control the operation of, data processing apparatus. The computer storage medium can be a machine-readable storage device, a machine-readable storage substrate, a random or serial access memory device, or a combination of one or more of them. Alternatively, or in addition, the program instructions can be encoded on an artificially generated propagated signal, e.g., a machine-generated electrical, optical, or electromagnetic signal, that is generated to encode information for transmission to suitable receiver apparatus for execution by a data processing apparatus.

The term "data processing apparatus" refers to data processing hardware and encompasses all kinds of apparatus, devices, and machines for processing data, including by way of example a programmable processor, a computer, or multiple processors or computers. The apparatus can also be, or further include, special purpose logic circuitry, e.g., an FPGA (field programmable gate array) or an ASIC (application specific integrated circuit). The apparatus can optionally include, in addition to hardware, code that creates an execution environment for computer programs, e.g., code that constitutes processor firmware, a protocol stack, a database management system, an operating system, or a combination of one or more of them.

A computer program, which may also be referred to or described as a program, software, a software application, an app, a module, a software module, a script, or code, can be written in any form of programming language, including compiled or interpreted languages, or declarative or procedural languages; and it can be deployed in any form, including as a stand-alone program or as a module, component, subroutine, or other unit suitable for use in a computing environment. A program may, but need not, correspond to a file in a file system. A program can be stored in a portion of a file that holds other programs or data, e.g., one or more scripts stored in a markup language document, in a single file dedicated to the program in question, or in multiple coordinated files, e.g., files that store one or more modules, sub programs, or portions of code. A computer program can be deployed to be executed on one computer or on multiple computers that are located at one site or distributed across multiple sites and interconnected by a data communication network.

In this specification, the term "database" is used broadly to refer to any collection of data: the data does not need to be structured in any particular way, or structured at all, and it can be stored on storage devices in one or more locations. Thus, for example, the index database can include multiple collections of data, each of which may be organized and accessed differently.

Similarly, in this specification the term "engine" is used broadly to refer to a software-based system, subsystem, or process that is programmed to perform one or more specific functions. Generally, an engine will be implemented as one or more software modules or components, installed on one or more computers in one or more locations. In some cases, one or more computers will be dedicated to a particular engine; in other cases, multiple engines can be installed and running on the same computer or computers.

The processes and logic flows described in this specification can be performed by one or more programmable computers executing one or more computer programs to perform functions by operating on input data and generating output. The processes and logic flows can also be performed by special purpose logic circuitry, e.g., an FPGA or an ASIC, or by a combination of special purpose logic circuitry and one or more programmed computers.

Computers suitable for the execution of a computer program can be based on general or special purpose microprocessors or both, or any other kind of central processing unit. Generally, a central processing unit will receive instructions and data from a read only memory or a random access memory or both. The essential elements of a computer are a central processing unit for performing or executing instructions and one or more memory devices for storing instructions and data. The central processing unit and the memory can be supplemented by, or incorporated in, special purpose logic circuitry. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device, e.g., a universal serial bus (USB) flash drive, to name just a few.

Computer readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto optical disks;

and CD ROM and DVD-ROM disks.

To provide for interaction with a user, embodiments of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's device in response to requests received from the web browser. Also, a computer can interact with a user by sending text messages or other forms of message to a personal device, e.g., a smartphone that is running a messaging application, and receiving responsive messages from the user in return.

Embodiments of the subject matter described in this specification can be implemented in a computing system that includes a back end component, e.g., as a data server, or that includes a middleware component, e.g., an application server, or that includes a front end component, e.g., a client computer having a graphical user interface, a web browser, or an app through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network (LAN) and a wide area network (WAN), e.g., the Internet.

The computing system can include clients and servers. A client and server are generally remote from each other and typically interact through a communication network. The relationship of client and server arises by virtue of computer programs running on the respective computers and having a client-server relationship to each other. In some embodiments, a server transmits data, e.g., an HTML page, to a user device, e.g., for purposes of displaying data to and receiving user input from a user interacting with the device, which acts as a client. Data generated at the user device, e.g., a result of the user interaction, can be received at the server from the device.

While this specification contains many specific implementation details, these should not be construed as limitations on the scope of any invention or on the scope of what may be claimed, but rather as descriptions of features that may be specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable sub combination. Moreover, although features may be described above as acting in certain combinations and even initially be claimed as such, one or more features from a claimed combination can in some cases be excised from the combination, and the claimed combination may be directed to a sub combination or variation of a sub combination.

Similarly, while operations are depicted in the drawings and recited in the claims in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system modules and components in the embodiments described above should not be understood as requiring such separation in all embodiments, and it should be understood that the described program components and systems can generally be integrated together in a single software product or packaged into multiple software products.

Particular embodiments of the subject matter have been described. Other embodiments are within the scope of the following claims. For example, the actions recited in the claims can be performed in a different order and still achieve desirable results. As one example, the processes depicted in the accompanying figures do not necessarily require the particular order shown, or sequential order, to achieve desirable results. In some cases, multitasking and parallel processing may be advantageous.

EMBODIMENTS

Although the present invention is defined in the claims, it should be understood that the present invention can also (alternatively) be defined in accordance with the following embodiments:

1. A medical monitoring system comprising:

a sensor apparatus configured to be attached to a user's body, wherein the sensor apparatus comprises:

one or more cardiac sensors, and
one or more acceleration sensors;

an electronic device communicatively coupled to the sensor apparatus, wherein the electronic device comprises one or more computer processors that are configured to:

cause the sensor apparatus to obtain, using the one or more cardiac sensors, cardiac data regarding the user during a first period of time,

cause the sensor apparatus to obtain, using the one or more acceleration sensors, acceleration data regarding the user during the first period of time,

determine, based on the cardiac data, first physiological data representing a cardiac activity of the user during the first period of time,

determine, based on the acceleration data, second physiological data representing a variation of the acceleration data during the period of time,

determine, based on the first physiological data and the second physiological data, a biomarker representing a health of the user, and

store a data structure representing the biomarker.

2. The medical monitoring system of embodiment 1, wherein the biomarker represents a functional capacity of the user.

3. The medical monitoring system of embodiment 1, wherein the biomarker represents a cardiopulmonary condition of the user.

4. The medical monitoring system of embodiment 1, wherein the sensor apparatus is configured to be attached to the user's chest.

5. The medical monitoring system of embodiment 1, wherein the first period of time is six minutes.

6. The medical monitoring system of embodiment 1, wherein the one or more cardiac sensors comprises an electrocardiogram (ECG) sensor.

7. The medical monitoring system of embodiment 1, wherein the first physiological data represents a number of heartbeats of the user during the first period of time.

8. The medical monitoring system of embodiment 7, wherein determining the first physiological data comprises: segmenting the cardiac data into a plurality of cardiac data segments, wherein each of the cardiac data segments represents a different respective heartbeat of the user.

9. The medical monitoring system of embodiment 8, wherein determining the first physiological data comprises: determining, based on the cardiac data segments, the number of heartbeats of the user during the first period of time.

10. The medical monitoring system of embodiment 1, wherein the acceleration data comprises a plurality of acceleration signals, wherein each of the acceleration signals represents acceleration in a different respective spatial dimension.

11. The medical monitoring system of embodiment 10, wherein determining the second physiological data comprises:

segmenting the acceleration data into a plurality of acceleration data segments, wherein each of the acceleration data segments represents a movement of the user during a different respective epoch of the first period of time, determining, for each of the epochs, a variation of the acceleration data during that epoch, and determining, based on the variation of the acceleration data during each of the epochs, the second physiological data.

12. The medical monitoring system of embodiment 11, wherein each of the epochs corresponds to a different respective time window during the first period of time.

13. The medical monitoring system of embodiment 12, wherein each of the time window is a 15 second window.

14. The medical monitoring system of embodiment 11, wherein determining the second physiological data comprises:

determining, for each of the epochs, a mean amplitude deviation of the acceleration data during that epoch, and summing the mean amplitude deviations of the acceleration data during each of the epochs.

15. The medical monitoring system of embodiment 14, wherein determining the second physiological data comprises: determining a log of the sum of the mean amplitude deviations of the acceleration data during each of the epochs.

16. The medical monitoring system of embodiment 15, wherein determining the second physiological data comprises: determining the second physiological data based on a regression model having the log of the sum of the mean amplitude deviations of the acceleration data as an input.

17. The medical monitoring system of embodiment 1, wherein determining the biomarker comprises determining a ratio of the first physiological data and the second physiological data.

18. The medical monitoring system of embodiment 1, wherein causing the biomarker to be presented to the user comprises:

determining a plurality of numerical ranges, wherein each of the numerical ranges corresponds to a different respective health rank,
determining that the biomarker is within a first numerical range of the plurality of numerical ranges, wherein the first numerical range corresponds to a first health rank, and causing the first health rank to be presented to the user.

19. The medical monitoring system of embodiment 1, wherein causing the biomarker to be presented to the user comprises:
presenting, using the display device, a graphical user interface to the user, wherein the graphical user interface comprises a graphical display element presenting the biomarker.

20. The medical monitoring system of embodiment 1, wherein causing the biomarker to be presented to the user comprises:
presenting, using the audio speaker, an audio notification to the user, wherein the audio notification indicates the biomarker.

21. The medical monitoring system of embodiment 1, wherein the one or more computer processors are further configured to:

determine, based on the biomarker, one or more recommendations for improving a health of the user, and
cause the one or more recommendations to be presented to the user.

22. The medical monitoring system of embodiment 1, wherein the one or more computer processors are further configured to:
instruct, using at least one of a display screen or an audio speaker, the user to walk during the first period of time.

23. The medical monitoring system of embodiment 1, wherein the one or more computer processors are further configured to:

cause the sensor apparatus to obtain the cardiac data and the acceleration data continuously during a second period of time, wherein the first period of time is a subset of the second period of time, and
select the first period of time based on at least one of the cardiac data or the acceleration data.

24. The medical monitoring system of embodiment 1, wherein the one or more computer processors are further configured to:
cause the biomarker to be presented to at least one of the user or an additional user.

25. A method comprising:

causing, using an electronic device, one or more cardiac sensors of a sensor apparatus to obtain cardiac data regarding a user during a first period of time;
causing, using the electronic device, one or more acceleration sensors of the sensor apparatus to obtain acceleration data regarding the user during the first period of time;

determining, by the electronic device and based on the cardiac data, first physiological data representing a cardiac activity of the user during the first period of time;

determining, by the electronic device and based on the acceleration data, second physiological data representing a variation of the acceleration data during the period of time;

determining, by the electronic device and based on the first physiological data and the second physiological data, a biomarker representing a health of the user; and

storing, by the electronic device, a data structure presenting the biomarker.

26. A system, comprising:

at least one processor; and

a memory communicatively coupled to the at least one processor, the memory storing instructions which, when executed by the at least one processor, cause the at least one processor to perform the method of embodiment 25.

27. One or more non-transitory computer-readable media storing instructions which, when executed by at least one processor, cause the at least one processor to perform the method of embodiment 25.

**Claims**

1. A medical monitoring system comprising:

   a sensor apparatus configured to be attached to a user's body, wherein the sensor apparatus comprises:

   one or more cardiac sensors, and
   one or more acceleration sensors;

   an electronic device communicatively coupled to the sensor apparatus, wherein the electronic device comprises one or more computer processors that are configured to:

   cause the sensor apparatus to obtain, using the one or more cardiac sensors, cardiac data regarding the user during a first period of time,
   cause the sensor apparatus to obtain, using the one or more acceleration sensors, acceleration data regarding the user during the first period of time,
   determine, based on the cardiac data, first physiological data representing a cardiac activity of the user during the first period of time,
   determine, based on the acceleration data, second physiological data representing a variation of the acceleration data during the period of time,
   determine, based on the first physiological data and the second physiological data, a biomarker representing a health of the user, and
   store a data structure representing the biomarker.

2. The medical monitoring system of claim 1, wherein

   the biomarker represents a functional capacity of the user; and/or
   the biomarker represents a cardiopulmonary condition of the user; and/or
   the sensor apparatus is configured to be attached to the user's chest; and/or
   the first period of time is six minutes; and/or
   the one or more cardiac sensors comprises an electrocardiogram, ECG, sensor.

3. The medical monitoring system of any one of claims 1 or 2, wherein the first physiological data represents a number of heartbeats of the user during the first period of time; optionally

   wherein determining the first physiological data comprises:
   segmenting the cardiac data into a plurality of cardiac data segments,
   wherein each of the cardiac data segments represents a different respective heartbeat of the user; further optionally
wherein determining the first physiological data comprises:
determining, based on the cardiac data segments, the number of heartbeats of the user during the first period of time.

4. The medical monitoring system of any one of claims 1 to 3, wherein the acceleration data comprises a plurality of acceleration signals, wherein each of the acceleration signals represents acceleration in a different respective spatial dimension.

5. The medical monitoring system of claim 4, wherein determining the second physiological data comprises:

   segmenting the acceleration data into a plurality of acceleration data segments, wherein each of the acceleration data segments represents a movement of the user during a different respective epoch of the first period of time,
   determining, for each of the epochs, a variation of the acceleration data during that epoch, and
   determining, based on the variation of the acceleration data during each of the epochs, the second physiological data.

6. The medical monitoring system of claim 5, wherein each of the epochs corresponds to a different respective time window during the first period of time, optionally wherein each of the time window is a 15 second window.

7. The medical monitoring system of any one of claims 5 or 6, wherein determining the second physiological data comprises:

   determining, for each of the epochs, a mean amplitude deviation of the acceleration data during that epoch, and
   summing the mean amplitude deviations of the acceleration data during each of the epochs; optionally
   wherein determining the second physiological data comprises:
   determining a log of the sum of the mean amplitude deviations of the acceleration data during each of the epochs;
   further optionally wherein determining the second physiological data comprises:
   determining the second physiological data based on a regression model having the log of the sum of the mean amplitude deviations of the acceleration data as an input.

8. The medical monitoring system of any one of claims 1 to 7, wherein determining the biomarker comprises determining a ratio of the first physiological data and the second physiological data.

9. The medical monitoring system of any one of claims 1 to 8, wherein causing the biomarker to be presented to the user comprises:

   determining a plurality of numerical ranges, wherein each of the numerical ranges corresponds to a different respective health rank,
   determining that the biomarker is within a first numerical range of the plurality of numerical ranges, wherein the first numerical range corresponds to a first health rank, and
   causing the first health rank to be presented to the user; and/or

   wherein causing the biomarker to be presented to the user comprises:

   presenting, using the display device, a graphical user interface to the user, wherein the graphical user interface comprises a graphical display element presenting the biomarker; and/or
   wherein causing the biomarker to be presented to the user comprises:
   presenting, using the audio speaker, an audio notification to the user,
   wherein the audio notification indicates the biomarker.

10. The medical monitoring system of any one of claims 1 to 9, wherein the one or more computer processors are further configured to:

   determine, based on the biomarker, one or more recommendations for improving a health of the user, and
   cause the one or more recommendations to be presented to the user; and/or
   wherein the one or more computer processors are further configured to:
   instruct, using at least one of a display screen or an audio speaker, the user to walk during the first period of time.

11. The medical monitoring system of any one of claims 1 to 10, wherein the one or more computer processors are further configured to:

cause the sensor apparatus to obtain the cardiac data and the acceleration data continuously during a second period of time, wherein the first period of time is a subset of the second period of time, and

select the first period of time based on at least one of the cardiac data or the acceleration data.

12. The medical monitoring system of any one of claims 1 to 11, wherein the one or more computer processors are further configured to:

cause the biomarker to be presented to at least one of the user or an additional user.

13. A method comprising:

causing, using an electronic device, one or more cardiac sensors of a sensor apparatus to obtain cardiac data regarding a user during a first period of time;

causing, using the electronic device, one or more acceleration sensors of the sensor apparatus to obtain acceleration data regarding the user during the first period of time;

determining, by the electronic device and based on the cardiac data, first physiological data representing a cardiac activity of the user during the first period of time;

determining, by the electronic device and based on the acceleration data, second physiological data representing a variation of the acceleration data during the period of time;

determining, by the electronic device and based on the first physiological data and the second physiological data, a biomarker representing a health of the user; and

storing, by the electronic device, a data structure presenting the biomarker.

14. A system, comprising:

at least one processor; and

a memory communicatively coupled to the at least one processor, the memory storing instructions which, when executed by the at least one processor, cause the at least one processor to perform the method of claim 13.

15. One or more non-transitory computer-readable media storing instructions which, when executed by at least one processor, cause the at least one processor to perform the method of claim 13.

Medical Monitoring System 100

**Electronic Device 120**

Database Module 122

Input Data 122a

Output Data 122b

Processing Rules 122c

Processing Module 126

Communications Module 124

User Interface Module 128

150

**Sensor Apparatus 110**

Cardiac Sensor(s) 112

Accelerometer(s) 114

Communications Module 116

118

FIG. 1

FIG. 2

FIG. 3

FIG. 4

FIG. 5

FIG. 6

FIG. 7

800

Cause one or more cardiac sensors of a sensor apparatus to obtain cardiac data regarding a
user during a first period of time
802

Cause one or more acceleration sensors of the sensor apparatus to obtain acceleration data
regarding the user during the first period of time
804

Determine, based on the cardiac data, first physiological data representing a cardiac activity
of the user during the first period of time
806

Determine, based on the acceleration data, second physiological data representing a variation
of the acceleration data during the period of time
808

Determine, based on the first physiological data and the second physiological data, a
biomarker representing a health of the user
810

Store a data structure presenting the biomarker
812

FIG. 8

FIG. 9

Europäisches
Patentamt

European
Patent Office

Office européen
des brevets

# EUROPEAN SEARCH REPORT

**Application Number**

EP 25 19 5135

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/077559 A1 (MC10 INC [US]; HUPPERT GILBERT LEE [US]) 28 May 2015 (2015-05-28) * 0052-0053, 0070-0073, 0084, 0088, 0091, 0097-0109, 0113, 0117; figures 1-26 * | 1-15 | INV. A61B5/0245 A61B5/11 A61B5/318 A61B5/349 A61B5/00 G16H20/30 |
| X | US 2024/194338 A1 (CHEN CHAO-WEN [TW] ET AL) 13 June 2024 (2024-06-13) * 0016, 0024-0046; figures 1, 2; tables 1-3 * | 1-15 | |
| X | US 2020/077951 A1 (NALLATHAMBI GABRIEL [US] ET AL) 12 March 2020 (2020-03-12) * 0020-0038; figures 1-6 * | 1-15 | |
| X | US 2016/058314 A1 (CHU CHI MING [TW]) 3 March 2016 (2016-03-03) * 0022-0057; figures 1-2 * | 1-15 | |
| A | RANTALAINEN TIMO ET AL: "Physical activity accumulation along the intensity spectrum differs between children and adults", EUROPEAN JOURNAL OF APPLIED PHYSIOLOGY, SPRINGER VERLAG, HEIDELBERG, DE, vol. 121, no. 9, 5 June 2021 (2021-06-05), pages 2563-2571, XP037536687, ISSN: 1439-6319, DOI: 10.1007/S00421-021-04731-3 [retrieved on 2021-06-05] * Abstract, "Methods", "Appendix 1" * | 1-15 | TECHNICAL FIELDS SEARCHED (IPC) A61B G16H |

-/--

The present search report has been drawn up for all claims

| Place of search | Date of completion of the search | Examiner |
|---|---|---|
| The Hague | 9 October 2025 | Lai, Marco |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................................................

& : member of the same patent family, corresponding document

EPO FORM 1503 03.82 (P04C01)

page 1 of 2

EUROPEAN SEARCH REPORT

Application Number

EP 25 19 5135

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHARLTON PETER H ET AL: "Accelerometry-Guided Inter-Beat-Interval Assessment from Wrist Photoplethysmography", 2023 COMPUTING IN CARDIOLOGY (CINC), CINC, vol. 50, 1 October 2023 (2023-10-01), pages 1-4, XP034506083, DOI: 10.22489/CINC.2023.046 [retrieved on 2023-12-26] * Abstract, section 2.3 "Assessing level of movement" * | 1-15 | |

TECHNICAL FIELDS
SEARCHED      (IPC)

The present search report has been drawn up for all claims

| Place of search | Date of completion of the search | Examiner |
|---|---|---|
| The Hague | 9 October 2025 | Lai, Marco |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 19 5135

This annex lists the patent family members relating to the patent documents cited in the above-mentioned European search report.
The members are as contained in the European Patent Office EDP file on
The European Patent Office is in no way liable for these particulars which are merely given for the purpose of information.

09-10-2025

| Patent document cited in search report | Publication date | Patent family member(s) | | Publication date |
|---|---|---|---|---|
| WO 2015077559 A1 | 28-05-2015 | CA | 2930740 A1 | 28-05-2015 |
| | | CN | 105813545 A | 27-07-2016 |
| | | EP | 3071096 A1 | 28-09-2016 |
| | | JP | 6711750 B2 | 17-06-2020 |
| | | JP | 7296344 B2 | 22-06-2023 |
| | | JP | 2017500093 A | 05-01-2017 |
| | | JP | 2020142118 A | 10-09-2020 |
| | | KR | 20160088882 A | 26-07-2016 |
| | | US | 2016287177 A1 | 06-10-2016 |
| | | US | 2018199884 A1 | 19-07-2018 |
| | | WO | 2015077559 A1 | 28-05-2015 |
| US 2024194338 A1 | 13-06-2024 | TW | 202423362 A | 16-06-2024 |
| | | US | 2024194338 A1 | 13-06-2024 |
| US 2020077951 A1 | 12-03-2020 | CN | 112654286 A | 13-04-2021 |
| | | EP | 3846679 A1 | 14-07-2021 |
| | | JP | 7625218 B2 | 03-02-2025 |
| | | JP | 2021536287 A | 27-12-2021 |
| | | US | 2020077951 A1 | 12-03-2020 |
| | | US | 2023100732 A1 | 30-03-2023 |
| | | WO | 2020051582 A1 | 12-03-2020 |
| US 2016058314 A1 | 03-03-2016 | TW | 201609051 A | 16-03-2016 |
| | | US | 2016058314 A1 | 03-03-2016 |

EPO FORM P0459

For more details about this annex : see Official Journal of the European Patent Office, No. 12/82